# EUROPEAN PATENT APPLICATION

(11) **EP 2 127 659 A1**
(43) Date of publication of application: **02.12.2009**
(21) Application number: 07846090.4
(22) Date of filing: 19.12.2007
(51) Int. Cl.: A61K 33/14, A61K 33/10, A61K 33/06, A61K 31/718, A61K 31/721, A61K 31/79, A61K 31/732, A61K 31/7004, A61K 31/047, A61P 17/02, A61P 41/00

(54) **USE OF HIGH OSMOTIC LIQUID COMPOSITION IN MANUFACTURING MEDICAMENT FOR ENHANCING WOUND HEALING**

(30) Priority: 28.12.2006 CN 200610148145
(71) Applicant: Zhao, Chaoying, Shanghai 200433 (CN)
(72) Inventor: Zhao, Chaoying, Shanghai 200433 (CN)
(74) Representative: Martin Santos, Victoria Sofia
(86) International application number: PCT/CN2007/071264
(87) International publication number: WO 2008/080336

(57) **Abstract**

A use of a hypertonic solution composition in manufacturing medicaments used during the perioperative period for promoting wound healing, said composition consists of 1.5%-6.9% (w/v) of one or more substances selected from sodium chloride, sodium bicarbonate, potassium chloride, magnesium sulfate, calcium chloride and calcium gluconate, 3%-18%(w/v) of one or more of the substances selected from hydroxyethyl starch, dextran, carboxymethyl starch, polyvinyl pyrrolidone and gelatin derivatives, and the remainder of conventional injection, provided that the amount of sodium chloride in the composition is not less than 1,5%(w/v), and the concentration of sodium ion is not more than the one equivalent to the concentration of sodium ion in 6.9% (w/v) sodium chloride solution. The hypertonic solution composition can be administered as transfusion before operation, during operation, after operation with the dose of 100-1500ml/person/day, which can promoter wound healing. The hypertonic solution composition is advantageous in terms of the safety and convenience of application. Said composition can applied to various operative wounds or trauma wound (surface) as well as anastomotic stoma.

## Description

### (1)TECHNICAL FIELD

The present invention relates to a use of a hypertonic solution composition in manufacturing medicaments for promoting wound healing.

### (2)BACKGROUND ART

Chirurgery plays an important role in modern medicine, wherein waiting for disconnecting until operative wound healing is part of performance after various surgeries. How to promote the heal of operative wound and anastomotic stoma has been a question confronted by chirurgery. Currently, the conventional measures refer to the supplement and infusion of isotonic solution after surgery, which often takes continuously several days for administration of 2500-3000 ml isotonic solution per day. However, the infusion of such a large amount of isotonic solution may influence circulating system in patients, increase burdens on the heart and lung, and therefore influence normal functions of these organs. It has been proven that the invention method of applying a hypertonic solution can promote wound (anastomotic stoma) healing. It is advantageous to substitute isotonic solution blood transfusion with part of hypertonic solution so that this substitution will reduce the amount of blood transfusion after surgery, for example 500 ml blood transfusion described in Example 1 can substantively substitute approximately 1000-1500 ml isotonic blood transfusion, and meanwhile maintain the function of circulating system as well as heart and lung, so as to promote the healing of operative wound (anastomotic stoma).

Further, Chinese Patent No. CN98108902.x, titled by "Pharmaceutical Compositions for the salvage and The Method for the Preparation thereof", filed on May 15, 1998 by the present applicant has disclosed a newly pharmaceutical composition, said composition is a hypertonic solution composition and can be widely applied to the treatment of various kinds of shock, cerebral trauma, burn, combined injury, cardiogenic shock caused by right ventricular infarction, hypotension caused by hemodialysis, biliary pancreatitis, operation patient, cardiovascular toxicity caused by narcotics and hepatic echinococcosis. However, this patent does not disclose the effect of said hypertonic solution composition on promoting wound healing. The whole content of this patent has been incorporated herein for reference.

### (3)SUMMARY OF THE INVENTION

The present applicant has attempted to use a hypertonic solution during the perioperative period and has obtained substantive effects. The results show that the use of hypertonic solution during delitescence period can alleviate inflammatory reactions such as hyperemia, exudation in the wound areas; promote the formation of granulation tissue during fibroplasia period, so as to result in the acceleration the wound healing.

An object of the present invention is to provide a use of a hypertonic solution composition in manufacturing medicaments for promoting wound healing.

The object of the present invention can be realized as follows. A use of a hypertonic solution composition in manufacturingmedicaments used during the perioperative period for promoting wound healing, said composition consists of 1.5%-6.9% (w/v) of one or more of the substances selected from the group consisting of sodium chloride, sodium bicarbonate, potassium chloride, magnesium sulfate, calcium chloride, calcium gluconate, calcium lactate, sodium lactate, sodium acetate and trihydroxymethyl aminomethane; 3%-18%(w/v) of one or more of the substances selected from the group consisting of hydroxyethyl starch, dextran, carboxymethyl starch, polyvinyl pyrrolidone, gelatin derivatives, polyglucose, glucose, fructose, lactose, glycerol, xylitol, sodium alginate, N-2 hydroxypropyl acrylamide, ethylene oxide-polypropylene glycol, pectin and penta-hydroxyethyl starch; and the balance amount of conventional injection, provided that the amount of sodium chloride in the composition is not less than 1.5%(w/v), and the concentration of sodium ion is not more than the one equivalent to the concentration of sodium ion in 6,9% (w/v) sodium chloride solution.

Preferably, the hypertonic solution composition comprises 4.2±0.2 g sodium chloride and 7.6±0.6g hydroxyethyl starch per 100 ml solution.

In the composition, said hydroxyethyl starch contains at least 10% hydroxyethyl starch with molecular weight of 25,000-45,000.

Said dextran has molecular weight of 40,000-230,000; carboxymethyl starch has molecular weight of 30,000-80,000; polyvinyl pyrrolidone has molecular weight of 5,000-700,000; polyglucose has molecular weight of 8,000-12,000; sodium alginate has molecular weight of 20,000-26,000; pectin has molecular weight of 20,000-40,000; penta-hydroxyethyl starch has molecular weight of 264,000.

Said gelatin derivatives have molecular weight of 20,000-35,000, and can be selected from urea-interlinkage gelatin, modified liquid gelatin, oxypolygelatin and degraded gelatin polypeptide.

Said conventional injection is selected from the group consisting of water for injection, physiological saline, balanced solution, glucose solution, sodium lactate solution, sodium acetate solution, trihydroxymethyl aminomethane solution, and saccharide saline.

The hypertonic solution composition of the present invention can be prepared by the following process: dissolving 3-18g of amount of one or more of the substances selected from hydroxyethyl starch, dextran, carboxymethyl starch, polyvinyl pyrrolidone, gelatin derivatives, polyglucose, glucose, fructose, lactose, glycerin, xylitol, sodium alginate, N-2-hydroxypropylacrylamide, ethylene epoxide-polypropylene glycol, pectin and pentahydroxyethylstarch in 100 ml of total volume of an injection or a mixture of several injections selected from water for injection, physiological saline, balanced solution, glucose solution, sodium lactate solution, sodium acetate solution, trihydroxymethyl aminomethane solution, and saccharide saline; then adding 1.5g sodium chloride and 0-5.4g of one or more of the substances selected from sodium chloride, sodium bicarbonate, potassium chloride, magnesium sulfate, calcium chloride, calcium gluconate, calcium lactate, sodium lactate, sodium acetate, and trihydroxymethyl aminomethane; with the proportion described above, then mixing, and dissolving, to obtain the hypertonic solution composition.

The hypertonic solution composition of the present invention can be administrated by transfusion during the perioperative period (before operation under anesthesia, during operation, or after operation), the composition can be transfused by drop. Preferably, each 500ml of the composition is administered within 20-60 min. The dosage depends on the ages and body weights of patients, usually the dosage for an adult is 250-1500 ml/person/day, while the dosage for a child can be reduced, such as 100ml/day or 50ml/day.

In an embodiment, said hypertonic solution composition may be administered by intravenous drip or in-bone drip during perioperative period.

The package containers for said hypertonic solution pharmaceutical composition of the present invention may be plastic bag, plastic bottle, glass bottle, glass ampoule. The volume of these containers can be 20ml, 50ml, 100ml, 250ml, 370ml or 500ml. Such package containers can be pre-installed or be attached with an intravenous (or in-bone) injection needle and/or transfusion tube, pressure device, transfusion pump.

### (4)BRIEF DESCRIPTION OF THE DRAWINGS

FIG. 1A-1E depict the tissue pathological observation results of group 3 of the hypertonic solution experiment groups described in Example 12.
FIG 2 depicts normal skin tissue and subcutaneous tissue of SD rats for comparison.
FIG 3A-3E depict the tissue pathological observation results of group 2 of the isotonic solution control groups described in Example 12.
FIG 4A-4E depict the tissue pathological observation results of group 4 of the hypertonic solution control groups described in Example 12.
FIG 5A-5E depict the tissue pathological observation results of group 5 of the hypertonic solution control groups described in Example 12.

### (5)DETAILED DESCRIPTION OF EMBODIMENTS

### EXAMPLE 1

Prepare according to the following proportions:

| | |
|---|---|
| hydroxyethyl starch | 7.6g |
| sodium chloride | 4.2g |
| water for injection added to | 100ml |

7.6g hydroxyethyl starch was dissolved in 100 ml of water for injection. 0.5g of activated charcoal was added, and the mixture was heated at 90°C for 15 min under stirring. After filtration, 4.2g sodium chloride (purity for medical application) was added, and dissolved with stirring. 0.5-1.0g activated charcoal was added, and the mixture was heated at 90°C for 10 min under stirring, filtered again and through 0.6-0.8µm micro-porous filter. The resulting filtrate was transferred into 20ml, 50ml, 100ml, 250ml, 370ml or 500ml glass ampoule, glass or plastic bottles (bags) for infusion, after sealing, the bottles or bags were sterilized under 1.05 kg/cm² at 121-123°C for 15-30 min, to obtain the hypertonic solution pharmaceutical composition of the present invention.

### EXAMPLE 2

Prepare according to the following proportion:

| | |
|---|---|
| hydroxyethyl starch | 8 g |
| sodium chloride | 4.1g |
| physiological saline | added to 100ml |

8g hydroxyethyl starch, 4.1g sodium chloride were dissolved in 100ml physiological saline, and the resulting mixture was adsorbed and bleached with activated charcoal. It was filtered, sealed and sterilized according to the method described in Example 1, to obtain the hypertonic solution pharmaceutical composition.

### EXAMPLE 3

Prepare according to the following proportion;

| | |
|---|---|
| sodium chloride | 5.1g |
| carboxymethyl starch | 18g |
| water for injection | added to 100ml |

18g carboxymethyl starch, 5.1g sodium chloride were dissolved in 100ml water for injection, and the resulting mixture was adsorbed and bleached with activated charcoal. Filtered, sealed and sterilized according to the method described in Example 1, to obtain the hypertonic solution pharmaceutical composition.

### EXAMPLE 4

Prepare according to the following proportion:

| | |
|---|---|
| hydroxyethyl starch | 6g |
| gelatin | 5g |
| sodium chloride | 5g |
| water for injection | added to 100ml |

The above-mentioned hydroxyethyl starch, gelatin and sodium chloride were dissolved in water for injection, and the resulting mixture was adsorbed and bleached with activated charcoal. Filtered, sealed and sterilized according to the method described in Example 1, to obtain the hypertonic solution pharmaceutical composition.

### EXAMPLE 5

Prepare according to the following proportion:

| | |
|---|---|
| hydroxyethyl starch | 6.5g |
| sodium chloride | 3g |
| sodium acetate | 2g |
| water for injection | added to 100ml |

The above-mentioned hydroxyethyl starch, sodium chloride and sodium acetate were dissolved water for injection, and the resulting mixture was adsorbed and bleached with activated charcoal. Filtered, sealed and sterilized according to the method described in Example 1, to obtain the hypertonic solution pharmaceutical composition.

### EXAMPLE 6

Prepare according to the following proportion:

| | |
|---|---|
| polyvinyl pyrrolidone | 12g |
| (produced by Bayer) | |
| sodium chloride | 2g |
| sodium bicarbonate | 4g |
| water for injection | added to 100ml |

The above-mentioned polyvinyl pyrrolidone, sodium chloride and sodium bicarbonate were dissolved in water for injection under stirring, and the resulting mixture was adsorbed and bleached with activated charcoal. Then it is filtered, sealed and sterilized according to the method described in Example 1, to obtain the hypertonic solution pharmaceutical composition.

### EXAMPLE 7

Prepare according to the following proportion:

| | |
|---|---|
| sodium alginate(produced by Nanning | 5g |
| Pharmaceutical Factory, Guangxi) | |
| sodium chloride | 1.5g |
| water for injection | added to 100ml |

The formulation was produced according to the method described in Example 1, to obtain the hypertonic solution pharmaceutical composition.

### EXAMPLE 8

Prepare according to the following proportion:

| | |
|---|---|
| pectin(produced by PLA No. | 3g |
| 185 Hospital) | |
| sodium chloride | 4g |
| sodium acetate solution(2%) | added to 100ml |

According to the method described in Example 1, pectin was dissolved in sodium acetate solution, and then sodium chloride was added until dissolution. The resulting solution was stirred, filtered, sealed and sterilized, to obtain the hypertonic solution pharmaceutical composition.

### EXAMPLE 9

Prepare according to the following proportion:

| | |
|---|---|
| polyglucose(produced by southwest | 7g |
| No.5 Pharmaceutical Factory, Chongqing) | |
| N-2- hydroxypropyl acrylamide | 2g |
| sodium chloride | 5.2g |
| water for injection | added to 100ml |

The formulation was produced according to the method described in Example 1, to obtain the hypertonic solution pharmaceutical composition.

### EXAMPLE 10

Prepare according to the following proportion:

| | |
|---|---|
| fructose(produced by Shanghai | 5g |
| No.2 Reagent Factory) | |
| xylitol(produced by Liaoyang Organic Chemical Plant) | 4g |
| sodium chloride | 4.8g |
| water for injection | added to 100ml |

The formulation was produced according to the method described in Example 1, to obtain the hypertonic solution pharmaceutical composition.

### EXAMPLE 11

Prepare according to the following proportion;

| | |
|---|---|
| glycerin | 2g |
| lactose(produced by Shanghai No.2 Reagent Factory) | 5g |
| sodium chloride | 5,3g |
| water for injection | added to 100ml |

The formulation was produced according to the method described in Example 1, to obtain the hypertonic solution pharmaceutical composition.

### EXAMPLE 12: Animal Experiment

### 1. Material and Method

### Animal

SD rats weighting 243-275g were provided by Shanghai Sub-center of National Rodent Experimental Animal Species Center. Feedstuff was provided by Shilin Biotechnic Co. Ltd., product standard was Q/TJCX1-2002. The SD rats were bred under regulation.

### Grouping

1. blank control group: the animals were not narcotized and operated;
2.isotonic solution control group: 0.9% sodium chloride solution were administered to the ratson the day of operation, the first day after operation and the second day after operation, respectively, with the dose of 8ml/kg/day. Such administrations were performed by tail vein infusion with the rate of 0.4 ml/kg/min;
3.hypertonic solution experimental group : 4.2% sodium chloride/7.6% hydroxyethyl starch solution(the product of Example 1) were administered to the rats on the day of operation, the first day after operation and the second day after operation, respectively, with the dose of 8ml/kg/day. Such administrations were performed by tail vein infusion with the rate of 0.4 ml/kg/min;
4.hypertonic solution control group: 1.5% sodium chloride/3.0% hydroxyethyl starch solution were administered to the ratsbefore operation under anesthesia, the first day after operation and the second day after operation, respectively, with the dose of 24ml/kg/day. Such administrations were performed by tail vein infusion with the rate of 1.2 ml/kg/min;
5.hypertonic solution control group: 6.9% sodium chloride/18% glucose solution were administered to the rats during operation under anesthesia, the first day after operation and the second day after operation, respectively, with the dose of 4ml/kg/day, Such administrations were performed by tail vein infusion with the rate of 0.2 ml/kg/min;

### Method

SD rats were anesthetized and peeled off hairs of lateral surface of the hind limb. The sterilization was done in accordance to conventional methods. After an operation cloth was spread on the experimental table, the rats' skins, the subcutaneous tissue, and muscular layer were cut to open, respectively. And then each layer was sutured till the skin layer. The experimental solution or the control solution was transfused according to the experimental requirements, after which the rats were put back and bred in the cage. At pre-determined time the animals were sacrificed and samples were made for pathological examines.

### 2. Results

There was no death report of the experimental animals during anesthesia, during operation, after operation and during transfusion. All of the wounds had achieved phase I healing, and no reports on wound infection. After pathological examines, there was no substantial difference of skin healing status between the hypertonic solution experimental group (Group 3) and the hypertonic solution control group (Goup 4, 5), while there was a significant difference of wound healing status between the hypertonic solution group and the isotonic solution control group. And therefore, compared with the isotonic solution control group (Group 2), the hypertonic solution experimental group (Group 3) was taken as an example to illustrate the pathological change status of wound healing.

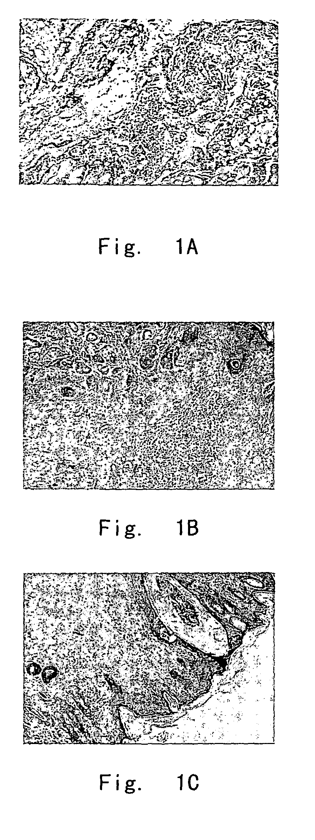

FIG 1A described the status of the hypertonic solution group (Group 3) on Day 3 after operation. There was moderate hyperemia, exudation, inflammatory cell infiltration and small necrosis in the wound tissue of the SD rats.

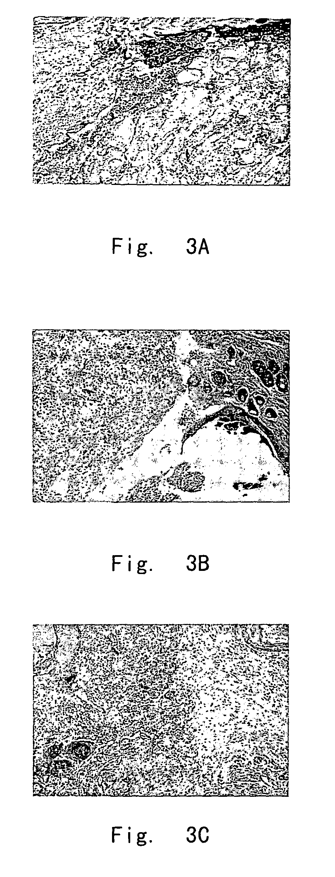

FIG 3A described the status of the isotonic solution group (Group 2) on Day 3 after operation. There was serious hyperemia, exudation, inflammatory cell infiltration and much necrosis in the wound tissue of the SD rats.

FIG 1B described the status of the hypertonic solution group (Group 3) on Day 6 after operation. There was granulation tissue proliferation and maturation in the wound of the SD rats.

FIG 3B described the status of the isotonic solution group (Group 2) on Day 6 after operation. There was granulation tissue formation, immaturation and visible necrosis in the wound of the SD rats.

FIG 1C described the status of the hypertonic solution group (Group 3) on Day 10 after operation, wherein the fibroblasts comprised granulation tissue as major part in the wound of the SD rats.

FIG 3C described the status of the isotonic solution group (Group 2) on Day 10 after operation, wherein the blood capillary comprised granulation tissue as major part in the wound of the SD rats.

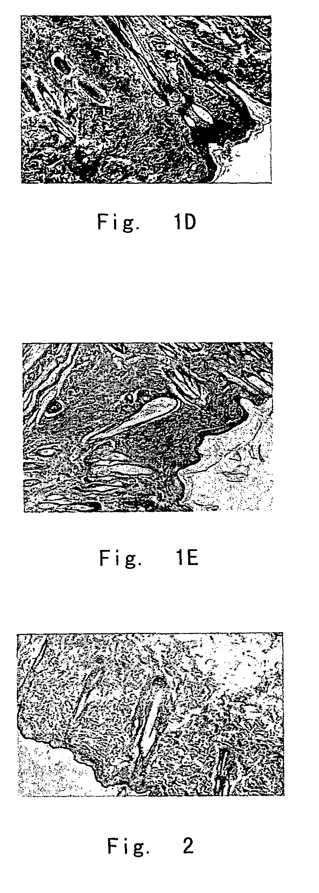

FIG 1D described the status of the hypertonic solution group (Group 3) on Day 15 after operation. The volume of scar was small in the wound of the SD rats.

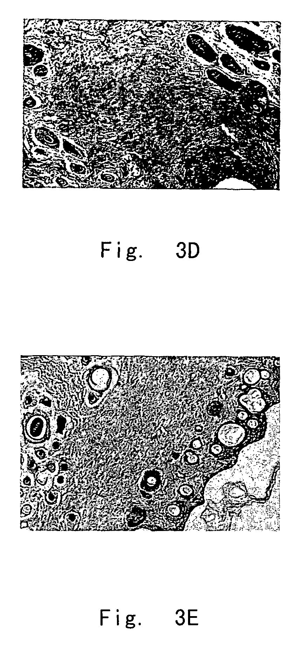

FIG 3D described the status of the isotonic solution group (Group 2) on Day 15 after operation. The volume of scar was large in the wound of the SD rats.

FIG 1E described the status of the hypertonic solution group (Group 3) on Day 20 after operation. The scar was essentially absorbed in the wound of the SD rats.

FIG 3E described the status of the isotonic solution group (Group 2) on Day 20 after operation. The absorption of scar in the wound of the SD rats was small.

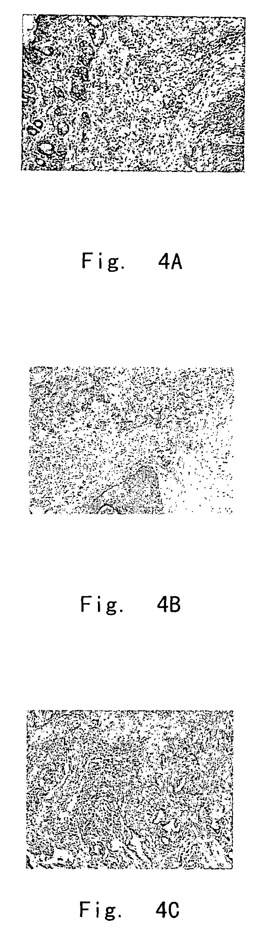

FIG 4A described the status of the hypertonic solution group (Group 4) on Day 3 after operation. There was moderate hyperemia, exudation, inflammatory cell infiltration and small necrosis in the wound tissue of the SD rats.

FIG 4B described the status of the hypertonic solution group (Group 4)on Day 6 after operation. There was granulation tissue proliferation and maturation in the wound of the SD rats.

FIG 4C described the status of the hypertonic solution group (Group 4) on Day 10 after operation. The fibroblasts comprised granulation tissue as major part in the wound of the SD rats.

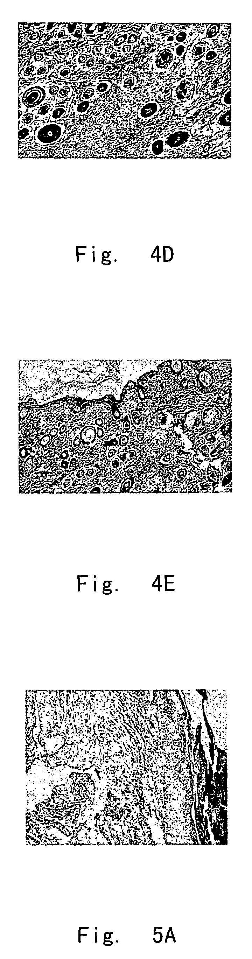

FIG 4D described the status of the hypertonic solution group (Group 4) on Day 15 after operation. The volume of scar was small in the wound of the SD rats.

FIG 4E described the status of the hypertonic solution group (Group 4) on Day 20 after operation. The scar was essentially absorbed in the wound of the SD rats.

FIG 5A described the status of the hypertonic solution group (Group 5) on Day 3 after operation. There was moderate hyperemia, exudation, inflammatory cell infiltration and small necrosis in the wound tissue of the SD rats.

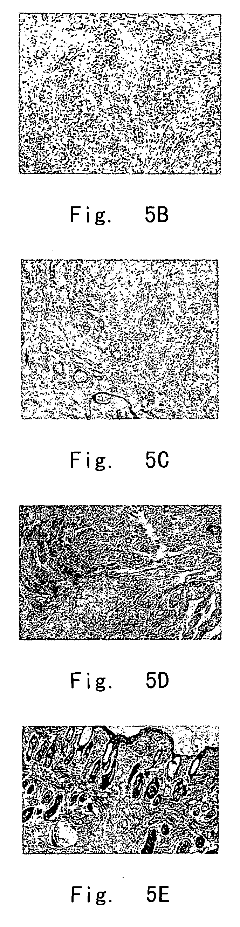

FIG 5B described the status of the hypertonic solution group (Group 5)on Day 6 after operation. There was granulation tissue proliferation and maturation in the wound of the SD rats.

FIG 5C described the status of the hypertonic solution group (Group 5) on Day 10 after operation. The fibroblasts comprised granulation tissue as major part in the wound of the SD rats.

FIG 5D described the status of the hypertonic solution group (Group 5) on Day 15 after operation. The volume of scar was small in the wound of the SD rats.

FIG 5E described the status of the hypertonic solution group (Group 5) on Day 20 after operation. The scar was essentially absorbed in the wound of the SD rats.

The experiment showed that the administration of the hypertonic solution did has a promotion effect on wound healing before operation under anesthesia, during operation, and after operation. The concrete manifestations are as follows: reducing the inflammatory reaction such as hyperemia, exudation in the wound area during delitescence period; promoting the formation of granulation tissue during the period of fibrous tissue proliferation, and therefore accelerating wound healing. Such change procedure can be found in the pathological tissue examination of FIG 1A-1E, 3A-3E, 4A-4E and 5A-5E in the specification, the comparison of the hypertonic solution and the isotonic solution was showed in table 1.

The experiment showed that the hypertonic solution containing 1.5%-6.9% sodium chloride had a promoting effect on wound healing, and therefore the concentration of hypertonic sodium chloride can be determined within this range. The applied dosages are 4ml/kg/day-24ml/kg/day, usually equivalent to 250ml/day-1500ml/day for an adult (this dosage can reduced properly for a child, for example 100ml/day or less); the rate of administration can not be too fast, and according to our previously experiment, for example, the administration period for 8ml/kg solution of Example 1 was 20min (or longer), and less than 60 min was considered to be appropriate (ref. Zhao Chaoying et. al., Chinese Practical Surgery, 2000, 20: 439).

**Table 1: The influence of administration of hypertonic solution and isotonic solution on wound healing**

| | | **hyperemia, exudation** | **inflammatory cell infiltration** | **necrosis** | **granulation tissue** | **Epidermis healing** | **scar** |
|---|---|---|---|---|---|---|---|
| Day 3 after operation | hypertonic solution group | moderate | moderate | small | formation, immaturation | none | none |
| | isotonic solution group | serious | serious | large | none | none | none |
| Day 6 after operation | hypertonic solution group | slight to moderate | moderate | none | proliferation, maturation | healing | none |
| | isotonic solution group | serious | serious | Exist | visible, immaturation | none | none |
| Day 10 after operation | hypertonic solution group | slight | slight | none | Fibroblast as major part | healing | none |
| | isotonic solution group | moderate | moderate to serious | none | blood capillary as major part | healing | none |
| Day 15 after operation | hypertonic solution group | none | none | none | none | healing | small |
| | isotonic solution group | slight to moderate | slight to moderate | none | none | healing | large |
| Day 20 after operation | hypertonic solution group | none | none | none | none | healing | small |
| | isotonic solution group | none | none | none | none | healing | large |

This animal experiments showed: the hypertonic solution pharmaceutical composition of the present invention to be transfused during perioperative period had a promoting effect on the healing of operative wounds. The experimental results showed: the effect of the hypertonic solution pharmaceutical composition was superior to the isotonic solution.

The use of the hypertonic solution pharmaceutical composition of the present invention during perioperative period is advantageous in terms of the safety and convenience of application. Said composition can applied to various operative wounds or trauma wound (surface) and anastomotic stoma.

## Claims

1. A use of a hypertonic solution composition in manufacturing medicaments used during the perioperative period for promoting the healing of operative wound or anastomotic stoma, said composition consists of 1.5%-6.9% (w/v) of one or more of the substances selected from the group consisting of sodium chloride, sodium bicarbonate, potassium chloride, magnesium sulfate, calcium chloride, calcium gluconate, calcium lactate, sodium lactate, sodium acetate and trihydroxymethyl aminomethane, 3%-18%(w/v) of one or more of the substances selected from the group consisting of hydroxyethyl starch, dextran, carboxymethyl starch, polyvinyl pyrrolidone, gelatin derivatives, polyglucose, glucose, fructose, lactose, glycerol, xylitol, sodium alginate, N-2-hydroxypropyl acrylamide, ethylene oxide-polypropylene glycol, pectin and penta-hydroxyethyl starch, and the balance amount of conventional injection, provided that the amount of sodium chloride in the composition is not less than 1,5%(w/v), and the concentration of sodium ion is not more than the one equivalent to the concentration of sodium ion in 6.9% (w/v) sodium chloride solution.

2. The use according to claim 1, wherein the hypertonic solution composition comprises 4.2±0.2 g sodium chloride and 7.6±0.6g hydroxyethyl starch per 100 ml solution.

3. The use according to claim 1 or 2, wherein the conventional injection is selected from the group consisting of water for injection, physiological saline, balanced solution, glucose solution, sodium lactate solution, sodium acetate solution, trihydroxymethyl aminomethane solution, and saccharide saline.

4. The use according to claim 1 or 2, wherein the hydroxyethyl starch contains at least 10% hydroxyethyl starch with molecular weight of 25,000-45,000.

5. The use according to claim 1, wherein the gelatin derivatives have molecular weight of 20,000-35,000, and are selected from the group consisting of urea-interlinkage gelatin, modified liquid gelatin, oxypolygelatin and degraded gelatin polypeptide.

6. The use according to claim 1, wherein the dextran has molecular weight of 40,000-230,000; carboxymethyl starch has molecular weight of 30,000-80,000; polyvinyl pyrrolidone has molecular weight of 5,000-700,000; polyglucose has molecular weight of 8,000-12,000; sodium alginate has molecular weight of 20,000-26,000; pectin has molecular weight of 20,000-40,000; penta-hydroxyethyl starch has molecular weight of 264,000.

7. The use according to claim 1 or 2, wherein the hypertonic solution composition is in the form of transfusion.

8. The use according to claim 1 or 2, wherein the hypertonic solution composition is intravenous drip composition or in-bone drip composition.

9. The use according to claim 1 or 2, wherein the package container for said hypertonic solution composition is plastic bag, plastic bottle, glass bottle or glass ampoule.

10. The use according to claim 9, wherein said package containeris pre-installed or attached with an intravenous or in-bone injection needle and/or blood transfusion tube, pressure device, transfusion pump.
